# FASCICULE DE BREVET EUROPEEN

(11) **EP 4 045 015 B1**
(45) Date de publication et mention de la délivrance du brevet: **06.03.2024**
(21) Numéro de dépôt: 20800706.2
(22) Date de dépôt: 16.10.2020
(51) Int. Cl.: A61K 9/19, A61K 47/02, A61K 47/18, A61K 47/26, A61K 9/08

(54) **NOUVEAU LYOPHILISAT D'HÉMISUCCINATE D'HYDROCORTISONE**
NEUARTIGES HYDROCORTISON-HEMISUCCINAT-LYOPHILISAT
NOVEL HYDROCORTISONE HEMISUCCINATE LYOPHILISATE

(30) Priorité: 18.10.2019 FR 1911670
(43) Date de publication de la demande: 24.08.2022
(73) Titulaire: Laboratoire Aguettant, 69007 Lyon (FR)
(72) Inventeur: REYNAUD, Fanny, 69007 LYON (FR); TONNAR, Jeff, 69007 LYON (FR)
(74) Mandataire: Germain Maureau
(86) Numéro de dépôt international: PCT/FR2020/051850
(87) Numéro de publication internationale: WO 2021/074542

(56) Documents cités:
- WO-A1-2018/078285
- CN-A- 103 371 978
- OLIVIER BAUD ET AL: "Effect of early low-dose hydrocortisone on survival without bronchopulmonary dysplasia in extremely preterm infants (PREMILOC): a double-blind, placebo-controlled, multicentre, randomised trial", THE LANCET, vol. 387, no. 10030, 1 avril 2016 (2016-04-01), pages 1827-1836, XP055685385, AMSTERDAM, NL ISSN: 0140-6736, DOI: 10.1016/S0140-6736(16)00202-6

## Description

La présente invention a pour objet un nouveau lyophilisat d'hémisuccinate d'hydrocortisone et son utilisation pour la préparation d'une solution injectable par voie intraveineuse ou parentérale utile pour la prévention ou le traitement de la dysplasie broncho-pulmonaire chez l'enfant prématuré.

La dysplasie broncho-pulmonaire (DBP) est une affection chronique des poumons fréquemment retrouvée chez les enfants prématurés ou grands prématurés (i.e. terme inférieur à 32 semaines d'aménorrhées) avec un faible poids à la naissance et nécessitant une ventilation mécanique dans le cadre d'un syndrome de détresse respiratoire aiguë. C'est l'une des principales complications et l'une des principales causes de mortalité et de morbidité chez les enfants prématurés ou grands prématurés. Chez le grand prématuré, le développement des alvéoles pulmonaires n'est pas terminé et la moindre agression peut altérer la maturation. C'est le cas, en particulier, lors d'une infection du liquide amniotique.

Chez le prématuré, il s'agit d'un facteur de risque de mortalité néonatale ou de séquelles neurologiques. Le risque de survenue de problèmes respiratoires durant l'enfance est également majoré. La fonction ventilation peut être altérée, même à l'âge adulte.

Les corticoïdes administrés par voie intraveineuse diminuent le risque de survenue d'une dysplasie broncho-pulmonaire chez les prématurés ou les grands prématurés. Cependant, les faibles doses devant être administrées nécessitent généralement de fortes dilutions des solutions mises sur le marché. Cette étape de préparation de la solution avant l'injection présente un risque important d'erreurs de dosage ou de contamination microbienne de la solution injectée.

Pour limiter ces risques, la Directive EMA/CHMP/QWP/805880/2012 Rev. 2 relative au développement pharmaceutique de médicaments pour usage pédiatrique émise par l'Agence Européenne du Médicament (EMA) préconise d'éviter ces dilutions en développant des formulations contenant les concentrations adéquates d'actif.

L'hémisuccinate d'hydrocortisone, également connu comme hydrogénosuccinate d'hydrocortisone, hydrocortisone succinate d'hydrogène ou simplement hydrocortisone succinate, est un corticostéroïde synthétique commercialisé dans de nombreux pays du monde en tant que médicament pour le traitement de l'insuffisance surrénale aiguë, de l'insuffisance surrénale transitoire du nouveau-né et de l'hyperplasie congénitale des surrénales avec syndrome de perte de sel (syndrome de Debré-Fibiger).

Cet actif est sujet à une hydrolyse rapide en milieu aqueux du fait de la liaison ester instable entre l'hydrocortisone et le succinate, si bien qu'il est à ce jour commercialisé sous la forme d'une poudre lyophilisée (ou lyophilisat) contenant 100mg ou 500mg d'hydrocortisone accompagné d'une solution de reconstitution.

Néanmoins, la posologie définie dans le cadre de la prévention ou du traitement d'une dysplasie broncho-pulmonaire chez les enfants prématurés ou grands-prématurés étant de 0,5mg d'hydrocortisone/kg/12 heures les 7 premiers jours puis 0,5 mg d'hydrocortisone/kg/24 heures pour les 3 jours suivants, il est nécessaire de diluer 50 fois (à l'aide d'une solution de glucose à 5%) la solution obtenue après reconstitution à partir du lyophilisat contenant 100 mg d'hydrocortisone et de la solution de reconstitution (2 ml) pour pouvoir l'administrer.

A la date de la présente invention, il n'existe pas de formulation permettant l'administration par voie intraveineuse (ou parentérale) de l'hydrocortisone à des enfants prématurés ou grands-prématurés dans les doses et volumes précités qui pourrait être reconstituée par simple ajout d'une solution de reconstitution et sans dilution subséquente.

Il existe donc un besoin avéré pour une telle composition. Cependant, la mise au point d'une telle composition est très complexe. En effet, il est connu que la lyophilisation est un procédé complexe où plusieurs paramètres doivent être considérés afin de garantir la conformité du produit. Bien que ce procédé permette d'améliorer significativement la stabilité de produits labiles en milieu aqueux, une attention particulière doit être apportée au choix de la formulation. En particulier, les paramètres tels que le pH, la nature et la quantité d'excipients dans la formulation ont un impact très important sur la stabilité du lyophilisat finalement préparé. En outre, des informations contradictoires au sujet de l'effet des différents excipients sur la stabilisation des principes actifs pharmaceutiques sont disponibles ce qui ne permet pas de déterminer de manière certaine une relation entre la structure d'un lyophilisat et sa stabilité, d'autant que la combinaison d'excipients peut, selon leur nature et leur proportion, affecter de façon inattendue la stabilité du lyophilisat. Enfin, les difficultés précitées sont encore accrues dans le cas d'un lyophilisat contenant de faibles (voire de très faibles) quantités de principe actif. En effet, une faible (voire une très faible) quantité d'actif dans un lyophilisat présuppose la présence d'un agent de charge en forte quantité, et donc de difficultés supplémentaires de mise au point d'une formulation stable et aisément resconstituable.

Il a pourtant été découvert, de façon totalement inattendue, que l'utilisation d'agents de charge particuliers combinée à l'ajout d'une certaine quantité d'agent tampon permettait de préparer un lyophilisat contenant de faibles (voire de très faibles) quantités d'hémisuccinate d'hydrocortisone stable dans le temps et facilement soluble dans un faible volume de solution de reconstitution en vue de préparer une solution prête à injectée par voie intraveineuse ou parentérale à des enfants prématurés ou grands-prématurés.

Ainsi, la présente invention a pour objet une poudre lyophilisée pour la préparation d'une solution injectable par voie intraveineuse ou parentérale comprenant :
- de 1% à 30% d'hémisuccinate d'hydrocortisone ;
- de 40% à 98% d'un agent de charge choisi parmi le mannitol, le tréhalose, le lactose, le sucrose, le raffinose ou le sorbitol ;
- de 1% à 30% d'un agent tampon.

La poudre lyophilisée selon l'invention est stable dans le temps lorsqu'elle est conservée à 5°C, voire même lorsqu'elle est conservée à température ambiante. En outre, elle permet la préparation de solution injectable par voie intraveineuse ou parentérale à des enfants prématurés (voire grands prématurés) pour prévenir et traiter la dysplasie broncho-pulmonaire par simple reconstitution, sans dilution subséquente.

Dans le cadre de la présente invention :
- on entend par « poudre lyophilisée » ou « lyophilisat » toute poudre ou poudre sèche contenant moins de 8% d'eau, de préférence moins de 5% d'eau, et préparée par toute méthode permettant d'atteindre un tel niveau d'humidité, en particulier la lyophilisation ;
- « hémisuccinate d'hydrocortisone » ou « hydrocortisone hémisuccinate » désigne le 11β,17α,21-trihydroxypregn-4-ene-3,20-dione 21-hemisuccinate, de numéro CAS 2203-97-6 (forme monohydrate) ou 83784-20-7 (forme anhydre) et de structure chimique : 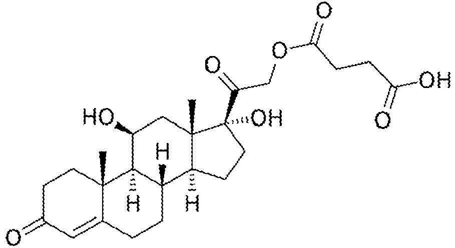 ainsi que ses sels (notamment son sel de sodium) pharmaceutiquement acceptables ;
- on entend par « sel pharmaceutiquement acceptable » d'un principe actif tout sel d'addition dudit principe actif avec un acide minéral ou organique par action d'un tel acide au sein d'un solvant organique ou aqueux tel qu'un alcool, une cétone, un éther ou un solvant chloré, et qui soit acceptable d'un point de vue pharmaceutique ; et
- on entend par « agent tampon » tout composé ou combinaison de composés permettant de maintenir le pH d'une solution à une valeur stable (+/- 1, de préférence +/- 0,5). Comme exemple d'agent tampon on peut notamment citer le citrate, le phosphate, le TRIS, l'histidine et l'HEPES ainsi que les sels associés.

De plus, dans le cadre de la présente invention, et sauf mention contraire, les proportions exprimées en % correspondent à des pourcentages massiques par rapport au poids total de l'entité considérée.

La présente invention a donc pour objet une poudre lyophilisée contenant de faibles (voire de très faibles) quantités d'hémisuccinate d'hydrocortisone, un agent de charge et un agent tampon tels que définis précédemment. Préférentiellement, la présente invention a pour objet une poudre lyophilisée telle que définie précédemment présentant les caractéristiques suivantes, prises seules ou en combinaison :
- la poudre lyophilisée contient de 2% à 20% d'hémisuccinate d'hydrocortisone, de préférence encore de 5% à 15% d'hémisuccinate d'hydrocortisone, et de façon tout à fait préférée de 8% à 12% d'hémisuccinate d'hydrocortisone ;
- l'agent de charge est choisi comme étant le mannitol ou le tréhalose, de préférence encore l'agent de charge est choisi comme étant le tréhalose ;
- la poudre lyophilisée contient de 50% à 95% d'agent de charge, de préférence de 60% à 90% d'agent de charge, et de façon tout à fait préférée de 75% à 85% d'agent de charge ;
- l'agent tampon est choisi comme étant le phosphate ou l'un de ses sels, de préférence l'agent tampon est choisi comme étant le phosphate sous forme de sel de sodium, de façon tout à fait préférée l'agent tampon est choisi comme étant un mélange de monosodium phosphate et de disodium phosphate ;
- la poudre lyophilisée contient de 2% à 20% d'agent tampon, de préférence de 5% à 15% d'agent tampon, et de façon tout à fait préférée de 8% à 12% d'agent tampon ;
- la poudre lyophilisée contient en outre un ou plusieurs composés permettant d'ajuster le pH tels que l'hydroxyde de sodium ou de l'acide orthophosphorique.

La poudre lyophilisée selon la présente invention peut être préparée selon tout procédé classiquement utilisé par l'homme de l'art. Comme exemple de procédé de préparation d'une poudre lyophilisée telle que définie précédemment, on peut notamment citer le procédé comprenant les étapes suivantes :
- ajout à température ambiante de l'agent tampon à 70% (v/v) à 90% (v/v) de l'eau totale nécessaire à la préparation de la solution avant lyophilisation ;
- ajout de l'hémisuccinate d'hydrocortisone ;
- ajustement du pH de la solution à une valeur variant de 7,0 à 8,0 de préférence 7,0 à 7,4 ;
- ajout de l'agent de charge ;
- ajout du reste de l'eau nécessaire ;
- filtration de la solution ;
- lyophilisation de la solution obtenue.

La poudre lyophilisée selon la présente invention peut donc être utilisée pour préparer une solution injectable par voie intraveineuse ou parentérale à des enfants prématurés ou grands-prématurés par simple ajout d'une solution de reconstitution.

Ainsi, la présente invention a également pour objet l'utilisation d'une poudre lyophilisée telle que définie précédemment pour la préparation d'une solution injectable par voie intraveineuse ou parentérale à des enfants prématurés ou grands-prématurés.

La présente invention a également pour objet un kit pour la préparation d'une solution injectable par voie intraveineuse ou parentérale à des enfants prématurés ou grands-prématurés comprenant :
- la poudre lyophilisée telle que définie précédemment ; et
- une solution de reconstitution.

De préférence, le kit selon la présente invention présente les caractéristiques suivantes, prises seules ou en combinaison :
- le kit contient de 1 mg à 50 mg de poudre lyophilisée, de préférence de 5 mg à 30 mg de poudre lyophilisée, de préférence encore de 10 mg à 20 mg de poudre lyophilisée, de façon tout à fait préférée 13 mg de poudre lyophilisée ;
- le kit contient de 0,1 ml à 10 ml de solution de reconstitution, de préférence de 0,2 ml à 5 ml de solution de reconstitution, de préférence encore de 0,5 ml à 2 ml de solution de reconstitution de façon tout à fait préférée 1 ml de solution de reconstitution ; et/ou
- la solution de reconstitution est choisie comme étant de l'eau ou un mélange d'eau et d'un agent osmotique, de préférence un mélange d'eau et de glucose.

La solution injectable par voie intraveineuse ou parentérale obtenue à partir du kit selon la présente invention peut donc être immédiatement injectée à un enfant prématuré (ou grand-prématuré) pour la prévention ou le traitement d'une dysplasie broncho-pulmonaire. Ainsi, la présente invention a également pour objet une solution injectable obtenue à partir du kit tel que décrit précédemment pour utilisation dans la prévention ou le traitement de la dysplasie broncho-pulmonaire chez l'enfant prématuré ou grand-prématuré.

Enfin, la présente invention a également pour objet une méthode de prévention ou de traitement de la dysplasie broncho-pulmonaire chez l'enfant prématuré ou grands-prématuré comprenant l'administration audit enfant d'une solution préparée à partir du kit décrit précédemment.

La présente invention est illustrée de manière non limitative par les exemples suivants.

### Exemple 1 - Préparation d'une poudre lyophilisée selon l'invention

### 1.1 Lyophilisat L1

Une poudre lyophilisée L1 dont la composition est rapportée dans le tableau 1 suivant a été préparée selon le procédé décrit ci-après.

**Tableau 1 - Lyophilisat L1**

| **Ingrédient** | **% (p/p)** |
|---|---|
| Hémisuccinate d'hydrocortisone | 10,5% |
| Tréhalose (agent de charge) | 79% |
| Phosphate sodium (agent tampon) | 1% |
| Phosphate disodium (agent tampon) | 9,5% |
| Hydroxyde de sodium (ajustement de pH) | qsp pH cible |
| Acide orthophosphorique (ajustement de pH) | qsp pH cible |

0,23 g de phosphate de sodium et 2,41 g de phosphate disodium sont solubilisés dans 800 ml d'eau pour préparation injectable à température ambiante. Après agitation et complète dissolution, 2,66 g d'hydrocortisone hémisuccinate sont ajoutés, toujours sous agitation. Le pH est ajusté à 7,4 jusqu'à ce que l'actif soit intégralement dissous. 20 g de tréhalose sont alors ajoutés à la solution. L'ensemble des poudres étant solubilisées, le pH est ajusté à la cible, soit 7,0 +/- 0,1, et le niveau d'eau est complété à 1 litre.

La lyophilisation est ensuite effectuée selon le cycle rapportée dans le tableau 2 suivant.

**Tableau 2 - Cycle de lyophilisation**

| **No.** | **Etape** | **Température** | **Pression** | **Durée** |
|---|---|---|---|---|
| 1 | Congélation | -45°C | Atmosphérique | 1 heure |
| 2 | Annealing | -25°C | Atmosphérique | 5 heures |
| 3 | Dessiccation primaire | -30°C | 70 µbar | 44 heures |
| 4 | Dessiccation secondaire | +30°C | 25 µbar | 10 heures |
| 5 | Bouchage sous azote | +5°C | 50 mbar | N/A |

### 1.2 Lyophilisat L2

Une poudre lyophilisée L2 dont la composition est rapportée dans le tableau 3 est préparée selon le procédé décrit dans l'exemple 1.1 ci-dessus.

**Tableau 3 - Lyophilisat L2**

| **Ingrédient** | **% (p/p)** |
|---|---|
| Hémisuccinate d'hydrocortisone | 17,4% |
| Mannitol (agent de charge) | 65,4% |
| Phosphate sodium (agent tampon) | 1,6% |
| Phosphate disodium (agent tampon) | 15,7% |
| Hydroxyde de sodium (ajustement de pH) | qsp pH cible |
| Acide orthophosphorique (ajustement de pH) | qsp pH cible |

### 1.3 Référence

Une poudre lyophilisée dite de référence « L_{ref}. » dont la composition est rapportée dans le tableau 4 est préparée selon le procédé décrit dans l'exemple 1.1 ci-dessus.

**Tableau 4 - Lyophilisat de référence L_{ref}.**

| **Ingrédient** | **% (p/p)** |
|---|---|
| Hémisuccinate d'hydrocortisone | 2% |
| Glucose (agent de charge) | 97% |
| Phosphate disodium (agent tampon) | 1% |
| Acide chlorhydrique | qsp pH cible |
| Hydroxyde de sodium | qsp pH cible |

### 2. Reconstitution de la solution d'injection

La reconstitution de la solution d'injection s'effectue en ajoutant 1 ml d'une solution de reconstitution eau/glucose (4,5/95,5) à un lyophilisat contenant 1 mg d'hydrocortisone (L1, L2 ou L_{ref.}). On obtient ainsi 1 ml de solution d'hydrocortisone base à 1 mg/ml.

Avec les lyophilisats L1 et L2, la reconstitution se fait immédiatement après une légère agitation du flacon pour homogénéiser la solution. La reconstitution ne présente donc aucune difficulté et s'effectue aussi facilement qu'avec les poudres lyophilisées actuellement sur le marché qui ne contiennent pas d'agent de charge.

La solution peut ensuite être prélevée pour être injectée à l'aide d'une seringue et d'une aiguille ou d'une seringue et d'un dispositif de transfert sans qu'aucune autre dilution ne soit nécessaire. Ainsi, les risques d'erreur de calcul ou de manipulation pouvant entraîner une erreur de dosage ainsi que les risques de contamination microbienne sont largement réduits en comparaison des produits actuellement sur le marché.

Au contraire, avec le lyophilisat L_{ref.}, on note une tendance marquée à collapser quelle que soit la température de stockage, ce qui rend la reconstitution plus difficile. Le temps de reconstitution est ainsi d'environ 30 secondes lorsque le lyophilisat est stocké à 5°C et peut aller jusqu'à 150 secondes lorsque le lyophilisat est stocké à 25°C ou 40°C. En outre, cette tendance marquée à collapser annonce une instabilité dans le temps du lyophilisat, quelle que soit la température de stockage.

### 3. Stabilité des lyophilisats

Les lyophilisats L1 et L2 ont été stockés aux températures de 5°C, 25°C et en conditions de stabilité accélérées à 40°C. Des analyses visant à déterminer le niveau d'impuretés (i.e. hydrocortisone libre), et donc la stabilité physico-chimique des formulations, ont été effectuées à T₀, T₀+1 mois, T₀+2 mois et T₀+3 mois.

Les résultats sont rapportés dans le tableau 5 suivant.

**Tableau 5 - Evolution de l'impureté hydrocortisone libre pour les formulations L1 et L2 à T₀, T₀+1 mois, T₀+2 mois et T₀+3 mois à 5°C, 25°C et 40°C**

| **Formulation / Conditions de stockage** | **Taux d'hydrocortisone libre (% (p/p))** | | | |
|---|---|---|---|---|
| | **T₀** | **T₀+1 mois** | **T₀+2 mois** | **T₀+3 mois** |
| L1 / 5°C | 0,6 | 0,8 | 0,8 | 0,8 |
| L1 / 25°C | 0,6 | 0,9 | 1,0 | 1,1 |
| L1 / 40°C | 0,6 | 1,4 | 1,9 | 2,4 |
| L2 / 5°C | 0,7 | 0,8 | 0,9 | 1,0 |
| L2 / 25°C | 0,7 | 1,8 | 3,0 | 4,0 |
| L2 / 40°C | 0,7 | **7,0** | **12,4** | **15,6** |

Le taux limite d'impureté toléré se situe à 6,7% (p/p).

On note que les lyophilisats L1 et L2 sont stables lorsqu'ils sont stockés à 5°C et à 25°C à T₀+3mois. Le lyophilisat L1 demeure même stable lorsqu'il est stocké en conditions de stockage accélérées à 40°C.

## Revendications

1. Poudre lyophilisée pour la préparation d'une solution injectable par voie intraveineuse ou parentérale comprenant :
- de 1% à 30% d'hémisuccinate d'hydrocortisone ;
- de 40% à 98% d'un agent de charge choisi parmi le mannitol, le tréhalose, le lactose, le sucrose, le raffinose ou le sorbitol ;
- de 1% à 30% d'un agent tampon.

2. Poudre lyophilisée selon la revendication 1, **caractérisée en ce qu'**elle contient 5% à 15% d'hémisuccinate d'hydrocortisone.

3. Poudre lyophilisée selon la revendication 1 ou 2, **caractérisée en ce que** l'agent de charge est choisi comme étant le mannitol ou le tréhalose.

4. Poudre lyophilisée selon l'une quelconque des revendications 1 à 3, **caractérisée en ce qu'**elle contient de 60% à 90% d'agent de charge.

5. Poudre lyophilisée selon l'une quelconque des revendications 1 à 4, **caractérisée en ce que** l'agent tampon est choisi comme étant le citrate, le phosphate, le TRIS, l'histidine ou l'HEPES ainsi que les sels associés.

6. Poudre lyophilisée selon l'une quelconque des revendications 1 à 5, **caractérisée en ce qu'**elle contient de 5% à 15% d'agent tampon.

7. Procédé de préparation d'une poudre lyophilisée selon l'une quelconque des revendications 1 à 6 comprenant les étapes suivantes :
- ajout à température ambiante de l'agent tampon à 70% (v/v) à 90% (v/v) de l'eau totale nécessaire à la préparation de la solution avant lyophilisation ;
- ajout de l'hémisuccinate d'hydrocortisone ;
- ajustement du pH de la solution à une valeur variant de 7 à 8 ;
- ajout de l'agent de charge ;
- ajout du reste de l'eau nécessaire ;
- filtration de la solution ;
- lyophilisation de la solution obtenue.

8. Utilisation d'une poudre lyophilisée selon l'une quelconque des revendications 1 à 6 pour la préparation d'une solution injectable par voie intraveineuse ou parentérale à des enfants prématurés ou grands-prématurés.

9. Kit pour la préparation d'une solution injectable par voie intraveineuse ou parentérale à des enfants prématurés ou grands-prématurés comprenant :
- la poudre lyophilisée selon l'une quelconque des revendications 1 à 6 ; et
- une solution de reconstitution.

10. Solution injectable par voie intraveineuse ou parentérale obtenue à partir du kit selon la revendication 9 pour utilisation dans la prévention ou le traitement de la dysplasie broncho-pulmonaire chez l'enfant prématuré ou grand-prématuré.

## Patentansprüche

1. Lyophilisiertes Pulver zur Herstellung einer intravenös oder parenteral injizierbaren Lösung, umfassend:
- 1 % bis 30 % Hydrocortison-Hemisuccinat;
- 40 % bis 98 % eines Füllmittels, ausgewählt aus Mannitol, Trehalose, Lactose, Sucrose, Raffinose oder Sorbitol;
- 1 % bis 30 % eines Puffermittels.

2. Lyophilisiertes Pulver nach Anspruch 1, **dadurch gekennzeichnet, dass** es 5 % bis 15 % Hydrocortison-Hemisuccinat enthält.

3. Lyophilisiertes Pulver nach Anspruch 1 oder 2, **dadurch gekennzeichnet, dass** das Füllmittel so ausgewählt ist, dass es Mannitol oder Trehalose ist.

4. Lyophilisiertes Pulver nach einem der Ansprüche 1 bis 3, **dadurch gekennzeichnet, dass** es 60 % bis 90 % Füllmittel enthält.

5. Lyophilisiertes Pulver nach einem der Ansprüche 1 bis 4, **dadurch gekennzeichnet, dass** das Puffermittel so ausgewählt ist, dass es Citrat, Phosphat, TRIS, Histidin oder HEPES sowie die zugehörigen Salze ist.

6. Lyophilisiertes Pulver nach einem der Ansprüche 1 bis 5, **dadurch gekennzeichnet, dass** es 5 % bis 15 % Puffermittel enthält.

7. Verfahren zur Herstellung eines lyophilisierten Pulvers nach einem der Ansprüche 1 bis 6, das die folgenden Schritte umfasst:
- Zugeben des Puffermittels bei Raumtemperatur zu 70 % (v/v) bis 90 % (v/v) des gesamten Wassers, das zur Herstellung der Lösung vor Lyophilisation benötigt wird;
- Zugeben des Hydrocortison-Hemisuccinats;
- Einstellen des pH-Werts der Lösung auf einen Wert, der von 7 bis 8 variiert;
- Zugeben des Füllmittels;
- Zugeben des Rests des benötigten Wassers;
- Filtrieren der Lösung;
- Lyophilisieren der erhaltenen Lösung.

8. Verwendung eines lyophilisierten Pulvers nach einem der Ansprüche 1 bis 6 zur Herstellung einer intravenös oder parenteral injizierbaren Lösung für frühgeborene oder extrem frühgeborene Kinder.

9. Set zur Herstellung einer intravenös oder parenteral injizierbaren Lösung für frühgeborene oder extrem frühgeborene Kinder, umfassend:
- das lyophilisierte Pulver nach einem der Ansprüche 1 bis 6; und
- eine Rekonstitutionslösung.

10. Intravenös oder parenteral injizierbare Lösung, die auf Grundlage des Sets nach Anspruch 9 erhalten wird, zur Verwendung bei der Vorbeugung oder der Behandlung von bronchopulmonaler Dysplasie bei frühgeborenen oder extrem frühgeborenen Kindern.

## Claims

1. A lyophilized powder for the preparation of a solution for intravenous or parenteral injection comprising:
- from 1% to 30% of hydrocortisone hemisuccinate;
- from 40% to 98% of a bulking agent chosen from mannitol, trehalose, lactose, sucrose, raffinose or sorbitol;
- from 1 % to 30% of a buffer agent.

2. The lyophilized powder according to claim 1, **characterized in that** it contains from 5% to 15% of hydrocortisone hemisuccinate.

3. The lyophilized powder according to claim 1 or 2, **characterized in that** the bulking agent is chosen as the mannitol or the trehalose.

4. The lyophilized powder according to any one of claims 1 to 3, **characterized in that** it contains from 60% to 90% of bulking agent.

5. The lyophilized powder according to any one of claims 1 to 4, **characterized in that** the buffer agent is chosen as the citrate, phosphate, TRIS, histidine or HEPES as well as the associated salts.

6. The lyophilized powder according to any one of claims 1 to 5, **characterized in that** it contains from 5% to 15% of buffer agent.

7. A method for preparing a lyophilized powder according to any one of claims 1 to 6 comprising the following steps:
- adding at room temperature the buffer agent at 70% (v/v) to 90% (v/v) of the total water necessary for the preparation of the solution before lyophilization;
- adding the hydrocortisone hemisuccinate;
- adjusting the pH of the solution to a value varying from 7 to 8;
- adding the bulking agent;
- adding the rest of the necessary water;
- filtering the solution;
- lyophilizing the obtained solution.

8. A use of a lyophilized powder according to any one of claims 1 to 6 for the preparation of a solution for intravenous or parenteral injection into premature or very premature children.

9. A kit for the preparation of a solution for intravenous or parenteral injection into premature or very premature children comprising:
- the lyophilized powder according to any one of claims 1 to 6; and
- a reconstitution solution.

10. A solution for intravenous or parenteral injection obtained from the kit according to claim 9 for use in the prevention or treatment of bronchopulmonary dysplasia in premature or very premature child.
